# EUROPEAN PATENT APPLICATION

(11) **EP 4 509 063 A1**
(43) Date of publication of application: **19.02.2025**
(21) Application number: 23788417.6
(22) Date of filing: 14.04.2023
(51) Int. Cl.: A61B 17/04, A61L 17/12

(54) **SUTURE FOR USE IN FLEXIBLE ENDOSCOPIC SURGERIES**

(30) Priority: 14.04.2022 JP 2022067190
(71) Applicant: National University Corporation Chiba University, Chiba-shi, Chiba 263-8522 (JP)
(72) Inventor: UESATO Masaya, Chiba-shi, Chiba 260-8670 (JP)
(74) Representative: Hoffmann Eitle
(86) International application number: PCT/JP2023/015127
(87) International publication number: WO 2023/200000

(57) **Abstract**

A suture 100 for use in flexible endoscopic surgeries including a suture body portion 10, one stop member 30 disposed at a center in a lengthwise direction of the suture body portion 10, and a plurality of protrusion portions 50 that obliquely protrude toward an opposite side to an insertion direction of the suture body portion 10, wherein the plurality of protrusion portions 50 include a plurality of first protrusion portions 51 that are disposed on one side, with the stop member 30 as a reference, in the lengthwise direction of the suture body portion 10, and protrude obliquely toward another side and outside of the suture body portion 10, and a plurality of second protrusion portions 52 that are disposed on the other side, with the stop member 30 as the reference, in the lengthwise direction of the suture body portion 10, and protrude obliquely toward the one side and outside of the suture body portion 10.

## Description

### TECHNICAL FIELD

The present invention relates to a suture for use in flexible endoscopic surgeries.

### BACKGROUND ART

An endoscopic surgery is a surgery that is performed inside a human or animal body while an image obtained by capturing an inside of the body with a lens disposed at a distal end of an endoscope is displayed on a monitor and confirmed. An endoscopic surgery tends to be able to decrease an opening area as compared with the conventional surgery that is performed by opening an abdomen or the like, and therefore, there are advantages that a patient experiences less postoperative pain, and recovers faster.

As an instrument for use in the conventional surgeries, for example, PTL1 has an object to provide a suture that is used in particular to fix a prosthesis to tissue, holds the suture in a predetermined place more reliably and accurately, and minimizes slippage of the suture and/or pledget that is used in combination with the suture, a suture system, a suturing method, a suturing system that simplifies a surgical technique and minimizes a possibility of a surgical error, and a suture that can pass through tissue and a suture ring more easily without causing damage, and discloses a surgical suture including an elongated core including a first leg portion and a second leg portion, and a stop that is projected from the elongated core between the first leg portion and the second leg portion. Note that it is described as preferable that the stop is incorporated to limit mobility of the pledget and/or improve performance of the suture.

Endoscopic surgeries are classified into a rigid endoscopic surgery and a flexible endoscopic surgery. The rigid endoscopic surgery is to open a plurality of small holes in a body surface of a patient, insert a rigid endoscope, a plurality of instruments and the like from the holes, and perform surgery of an inside of a body of the patient. The flexible endoscopic surgery is to use an instrument inserted and removed through a forceps hole disposed at a distal end of a flexible endoscope to perform surgery of the inside of the body of a patient, while confirming the image captured by the lens disposed in the distal end of the flexible endoscope.

As an instrument used in endoscopic surgery, for example, PTL2 discloses a suture for use in endoscopic surgery composed of a first portion extending along a predetermined length from one end and a second portion except for the first portion, characterized in that the first portion and the second portion have different colors from each other, and describes that the suture that can easily perform suturing in endoscopic surgeries can be provided.

### CITATION LIST

### PATENT LITERATURE

PTL1: JP 2013-502990 A
PTL2: JP 2015-58110 A

### SUMMARY OF INVENTION

### TECHNICAL PROBLEM

The surgery suture described in PTL1 is not intended or optimized for use in performing surgery of an inside of a body of a patient (intestinal tract, for example) by using a rigid or flexible endoscope. Further, in a flexible endoscopic surgery, surgery on affected area of a patient is performed by using only an instrument inserted and removed from a forceps hole disposed in a distal end of a flexible endoscope. Conventional sutures (suture disclosed in PTL2 and the like, for example) that are used in flexible endoscopic surgeries are generally used only for suturing surface layers of the mucous membranes and the like of stomachs and intestines. Therefore, when the conventional flexible endoscopic surgery suture is used to close an opening portion formed by incision or the like of human or animal tissue, for example, there has been a problem that it is difficult to continue inserting the suture for use in flexible endoscopic surgeries so that the edge of the opening portion faces an inside of the opening portion (direction that is normally visible through a lens of a flexible endoscope), and it is difficult to completely close the opening portion.

Further, unlike ordinary surgeries using no endoscopes, or rigid endoscopic surgeries, in the flexible endoscopic surgeries, it is difficult to suture while gripping, or pressing the affected area or the like. Accordingly, in flexible endoscopic surgery, there has been a problem that a suturing time tends to be long.

An object of the present invention is to provide a suture for use in flexible endoscopic surgeries that can easily and completely close an opening portion of human or animal tissue, and can shorten a suturing time.

### SOLUTION TO PROBLEM

The present inventor found that the above-described problem can be solved by adopting the following configuration, and completed the invention.

That is to say, the present invention is as follows.
<1> A suture for use in flexible endoscopic surgeries for suturing an opening portion of human or animal tissue including a suture body portion, one stop member that is disposed at a center in a lengthwise direction of the suture body portion and prevents the suture body portion from passing through, and a plurality of protrusion portions that obliquely protrude toward an opposite side to an insertion direction of the suture body portion, wherein the plurality of protrusion portions include a plurality of first protrusion portions that are disposed on one side, with the stop member as a reference, in the lengthwise direction of the suture body portion, and protrude obliquely toward another side and outside of the suture body portion, and a plurality of second protrusion portions that are disposed on the other side, with the stop member as the reference, in the lengthwise direction of the suture body portion, and protrude obliquely toward the one side and outside of the suture body portion.
<2> The suture for use in flexible endoscopic surgeries according to <1> described above, wherein both ends of the suture body portion are inserted from an outer wall side to an inner wall side of tissue in a vicinity of the opening portion.
<3> The suture for use in flexible endoscopic surgeries according to <1> or <2> described above, wherein one end of the suture body portion is inserted from an outer wall side to an inner wall side of tissue in a vicinity of the opening portion where an opening diameter of the opening portion is maximum, and another end of the suture body portion is inserted from an outer wall side to an inner wall side of tissue located on an opposite side to the tissue of the opening portion where the one end of the suture body portion is inserted.
<4> The suture for use in flexible endoscopic surgeries according to any one of <1> to <3> described above, wherein the stop member holds the opening portion in a closed state on an outer wall side in cooperation with the plurality of protrusion portions.
<5> The suture for use in flexible endoscopic surgeries according to any one of <1> to <4> described above, wherein the suture body portion includes a first suture piece having the plurality of protrusion portions, and a second suture piece having the plurality of protrusion portions, and the stop member is disposed at a joining portion of the first suture piece and the second suture piece.
<6> The suture for use in flexible endoscopic surgeries according to any one of <1>to <5> described above, wherein the stop member has a spherical shape.
<7> The suture for use in flexible endoscopic surgeries according to any one of <1> to <5> described above, wherein the stop member is plate-shaped.
<8> The suture for use in flexible endoscopic surgeries according to any one of <1> to <5> described above, wherein the stop member has a coil shape.
<9> The suture for use in flexible endoscopic surgeries according to <1> to <5> described above, wherein the stop member is a knot formed by tying the suture body portion.
<10> The suture for use in flexible endoscopic surgeries according to any one of <1> to <5> described above, wherein the stop member is a clip that can hold the suture body portion.
<11> The suture for use in flexible endoscopic surgeries according to any one of <1> to <10> described above, wherein the suture is made of a bioabsorbable material.
<12> A suture unit for use in flexible endoscopic surgeries using the suture for use in flexible endoscopic surgeries according to any one of <1> to <11> described above, including needles at both ends of the suture body portion.

### ADVANTAGEOUS EFFECTS OF INVENTION

According to the present invention, there can be provided a suture for use in flexible endoscopic surgeries that can easily and completely close an opening portion of human or animal tissue, and can shorten a suturing time thereof.

### BRIEF DESCRIPTION OF DRAWINGS

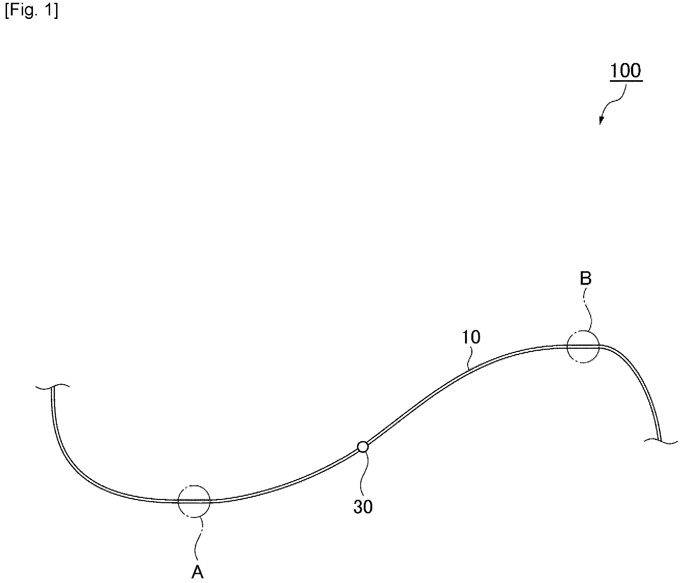

[Fig. 1] Fig. 1 is a view for explaining a configuration example of a suture for use in flexible endoscopic surgeries according to a first embodiment.

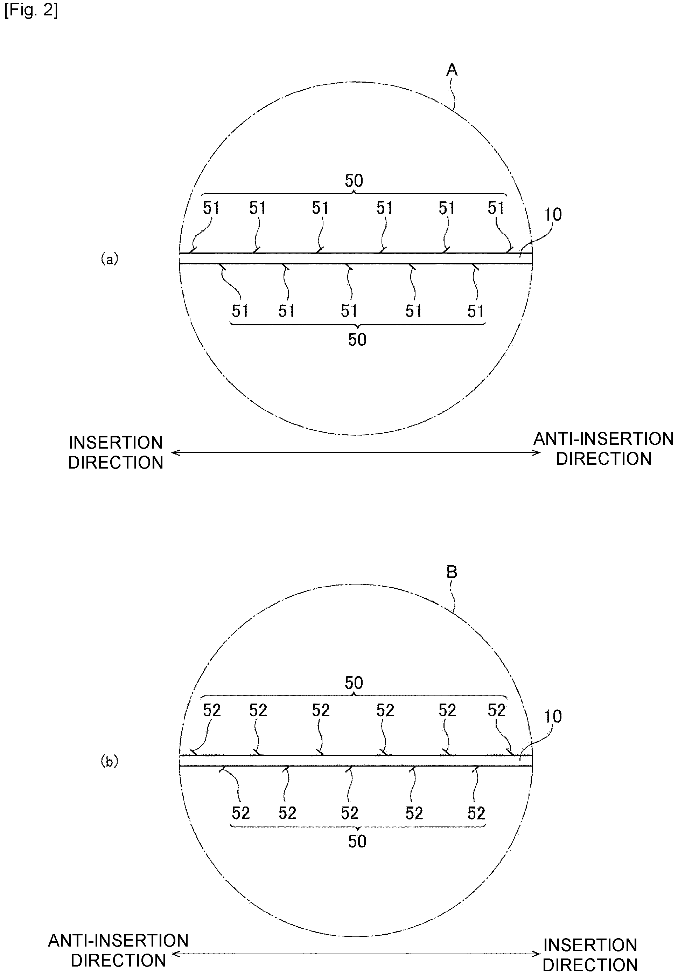

[Fig. 2] Fig. 2 is a partially enlarged view of the suture for use in flexible endoscopic surgeries according to the first embodiment, a partial view (a) in Fig. 2 is a partially enlarged view of a spot shown by reference sign A in Fig. 1, and a partial view (b) in Fig. 2 is a partially enlarged view of a spot shown by reference sign B in Fig. 1.

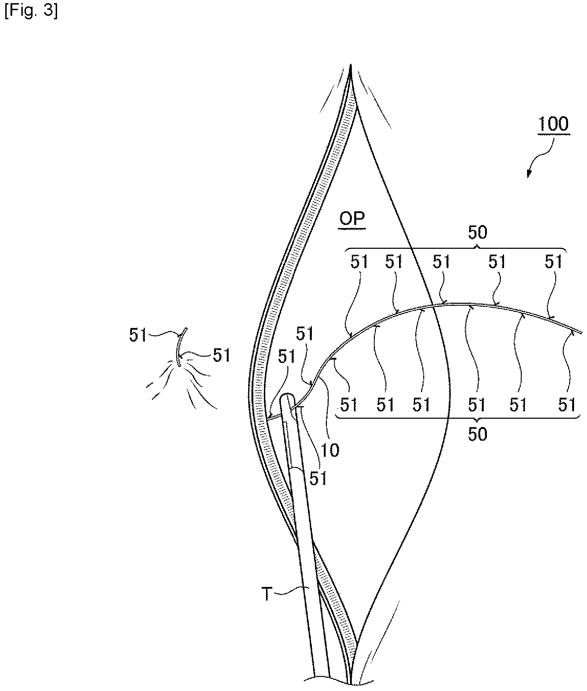

[Fig. 3] Fig. 3 is a view explaining a state in which one end of the suture for use in flexible endoscopic surgeries according to the first embodiment is inserted from an outer wall side to an inner wall side of tissue in a vicinity of an opening portion.

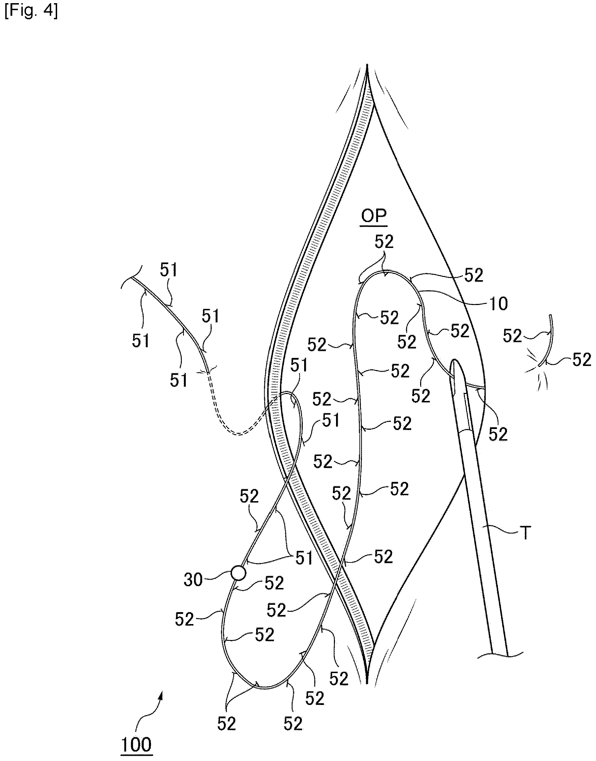

[Fig. 4] Fig. 4 is a view explaining a state in which after the one end of the suture for use in flexible endoscopic surgeries according to the first embodiment is inserted from the outer wall side to the inner wall side of the tissue in the vicinity of the opening portion, the other end of the suture for use in flexible endoscopic surgeries is inserted into tissue located on an opposite side to the tissue of the opening portion where the one end of the suture for use in flexible endoscopic surgeries is inserted from an outer wall side to an inner wall side.

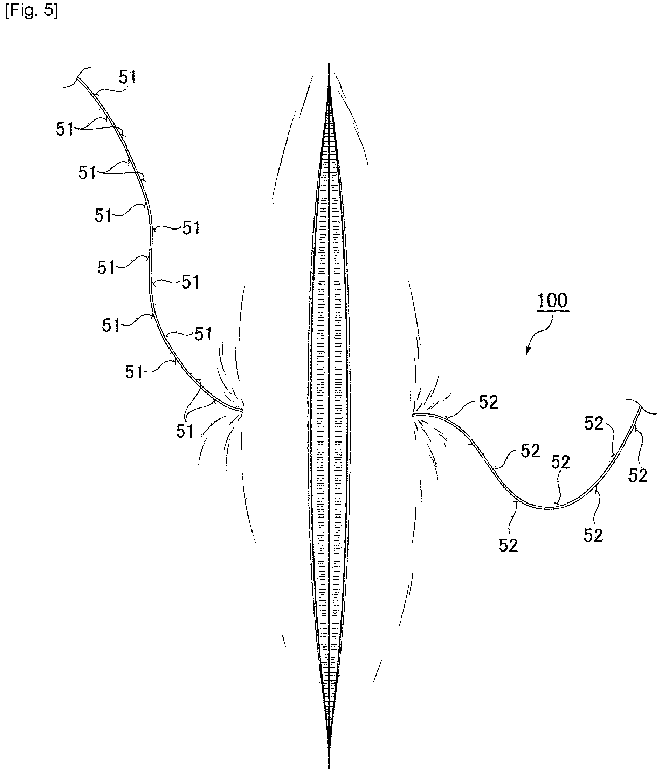

[Fig. 5] Fig. 5 is a view for explaining a state after both the ends of the suture for use in flexible endoscopic surgeries according to the first embodiment are inserted from the outer wall side to the inner wall side of the tissue in the vicinity of the opening portion.

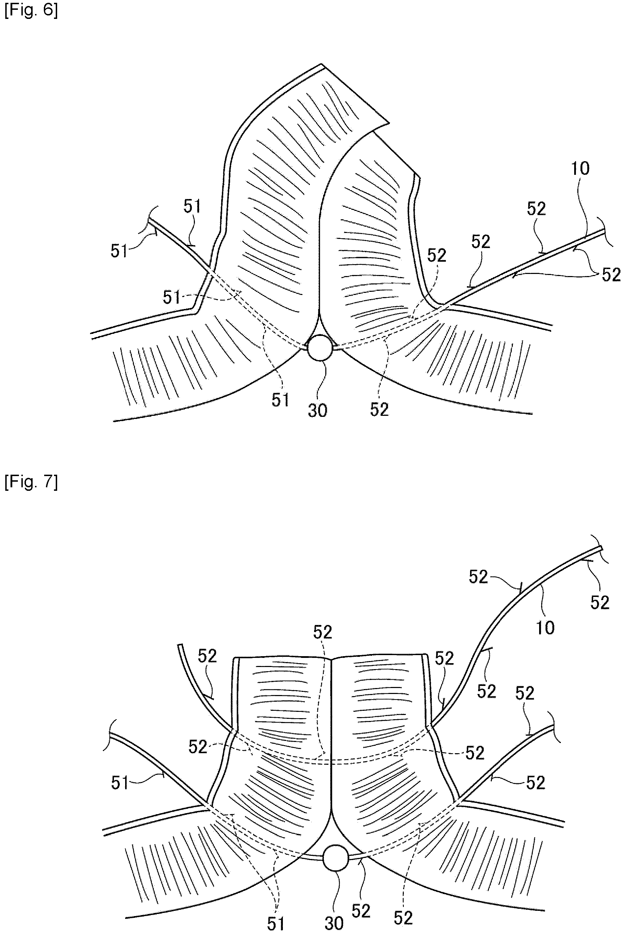

[Fig. 6] Fig. 6 is a sectional view for explaining a state in which after both ends of the suture for use in flexible endoscopic surgeries according to the first embodiment are inserted from the outer wall side to the inner wall side of the tissue in the vicinity of the opening portion, tension is further applied from one side of the suture for use in flexible endoscopic surgeries.
[Fig. 7] Fig. 7 is a sectional view for explaining a state in which one end of the suture for use in flexible endoscopic surgeries is inserted into tissue that is raised by inserting both the ends of the suture for use in flexible endoscopic surgeries according to the first embodiment from the outer wall side to the inner wall side of the tissue in the vicinity of the opening portion and applying tension from one side.

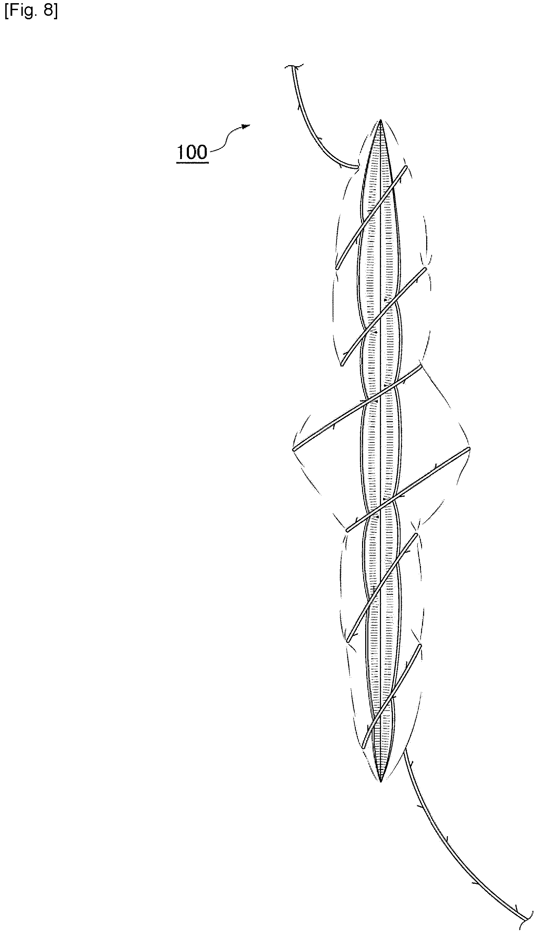

[Fig. 8] Fig. 8 is a view for explaining a state in which suturing of the opening portion is completed by the suture for use in flexible endoscopic surgeries according to the first embodiment.

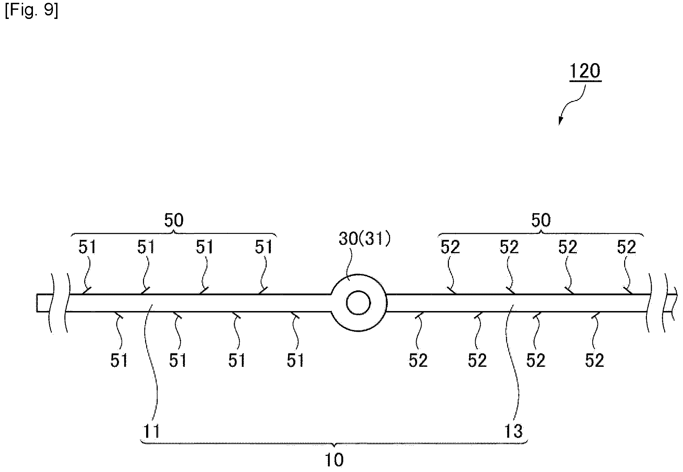

[Fig. 9] Fig. 9 is a view for explaining a configuration example of a suture for use in flexible endoscopic surgeries according to a second embodiment.

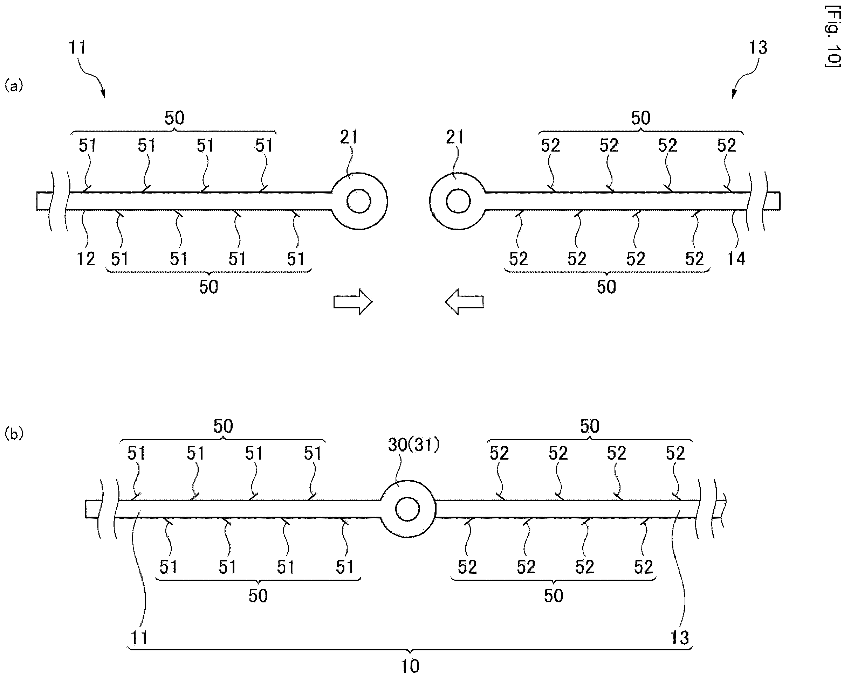

[Fig. 10] Fig. 10 is a view showing an example of a method for manufacturing a suture for use in flexible endoscopic surgeries according to a second embodiment, a partial view (a) in Fig. 10 is a view illustrating configurations of a first suture piece and a second suture piece, and a partial view (b) in Fig. 10 is a view illustrating a state in which the first suture piece and the second suture piece are joined to each other.

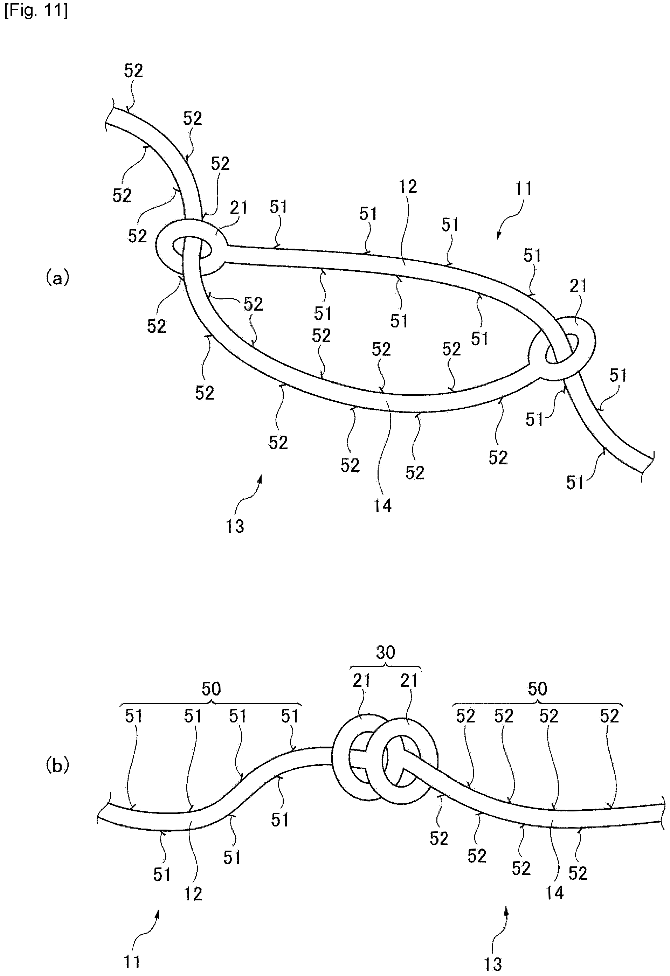

[Fig. 11] Fig. 11 is a view showing an example of another method for manufacturing a suture for use in flexible endoscopic surgeries according to the second embodiment, a partial view (a) in Fig. 11 is a view illustrating a state in which a second suture piece is inserted into a large diameter portion of a first suture piece, and a first suture is inserted into a large diameter portion of the second suture piece, and a partial view (b) in Fig 11 is a view illustrating a state in which the large diameter portion of the first suture piece and the large diameter portion of the second suture piece are joined to each other.

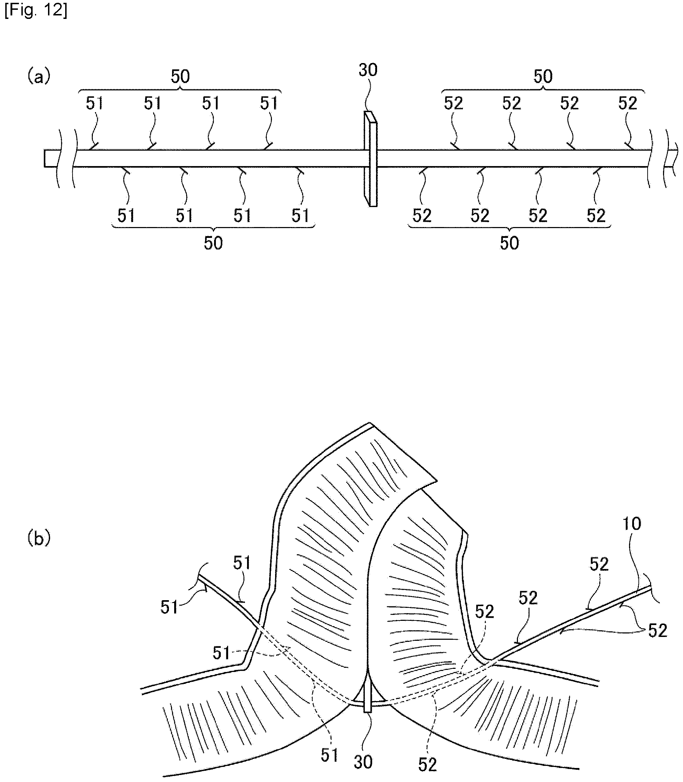

[Fig. 12] Fig. 12 is a view for explaining a stop member according to a modification, a partial view (a) in Fig. 12 is a view for explaining a configuration example of the stop member according to the modification, and a partial view (b) in Fig. 12 is a sectional view for explaining a state in which after both ends of a suture body portion are inserted from an outer wall side to an inner wall side of tissue in a vicinity of an opening portion, tension is further applied from one side of the suture body portion.

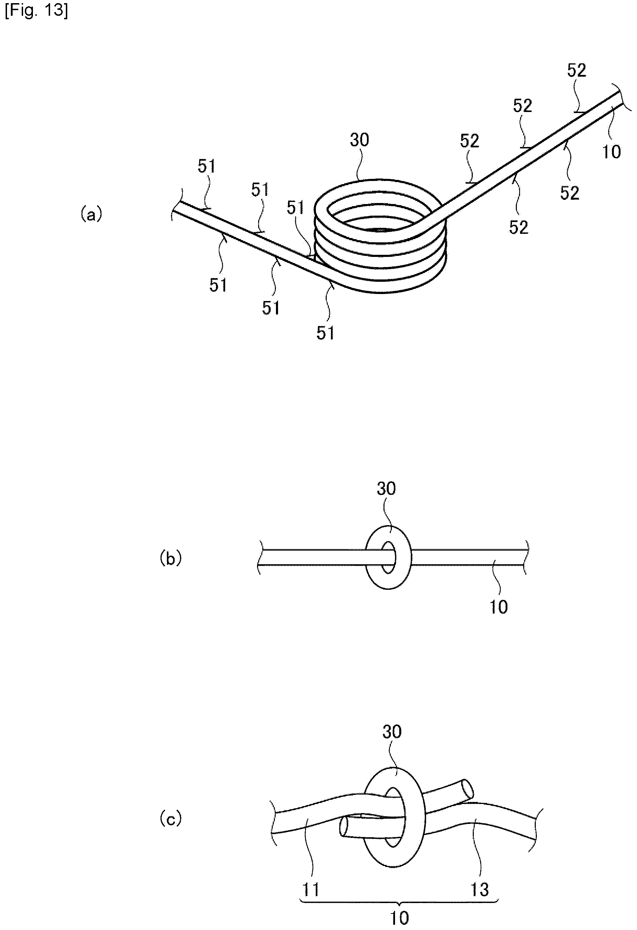

[Fig. 13] Fig. 13 is a view for explaining a configuration example of a stop member according to a modification.

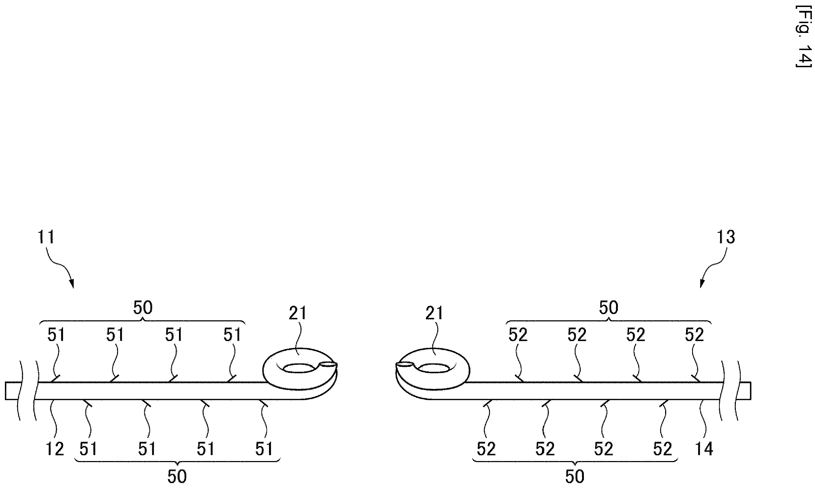

[Fig. 14] Fig. 14 is a view for explaining configuration examples of a first suture piece and a second suture piece according to a modification.

### DESCRIPTION OF EMBODIMENTS

Hereinafter, preferred embodiments for carrying out the present invention will be described using the drawings. Note that the following embodiments are not intended to limit the invention according to respective claims, and all combinations of features described in the embodiments are not essential to the solution of the invention.

First, a configuration example of a suture 100 for use in flexible endoscopic surgeries according to a first embodiment will be described using Fig. 1 and Fig. 2. Here, Fig. 1 is a view for explaining a configuration example of the suture for use in flexible endoscopic surgeries according to the first embodiment. Fig. 2 is a partially enlarged view of the suture for use in flexible endoscopic surgeries according to the first embodiment, a partial view (a) in Fig. 2 is a partially enlarged view of a spot shown by reference sign A in Fig. 1, and a partial view (b) in Fig. 2 is a partially enlarged view of a spot shown by reference sign B in Fig. 1.

### [Suture 100 for Use in Flexible Endoscopic Surgeries According to First Embodiment]

As shown in Fig. 1 and Fig. 2, a suture 100 for use in flexible endoscopic surgeries according to the first embodiment is for suturing an opening portion of human or animal tissue, and includes a suture body portion 10, one stop member 30 that is disposed at a center in a lengthwise direction of the suture body portion 10 and prevents the suture body portion 10 from passing through, and a plurality of protrusion portions 50 that protrude obliquely toward an opposite side (anti-insertion direction) to an insertion direction of the suture body portion 10, and the plurality of protrusion portions 50 include a plurality of first protrusion portions 51 that are disposed on one side, with the stop member 30 as a reference, in the lengthwise direction of the suture body portion 10, and protrude obliquely toward the other side and outside of the suture body portion 10, and a plurality of second protrusion portions 52 that are disposed on the other side, with the stop member 30 as the reference, in the lengthwise direction of the suture body portion 10, and protrude obliquely toward the one side and outside of the suture body portion 10.

In the description, the opening portion of human or animal tissue (hereinafter, also simply referred to as an opening portion) refers to a portion that is opened and a target to be sutured in the tissue in a human or animal body, and often generally refers to an opening portion of a digestive tract inner wall under a flexible endoscope although a shape, a size and the like thereof is not particularly limited.

As shown in Fig. 1, the suture body portion 10 is a body portion of the suture 100 for use in flexible endoscopic surgeries. The suture body portion 10 may be composed of one thread, or may be composed of a combination of two threads as described later. Further, the suture body portion 10 may be made of multiple overlapping threads such as double threads. In addition to the suture body portion 10, components of the suture 100 for use in flexible endoscopic surgeries are preferably made of bioabsorbable materials that do not require removal and do not leave any foreign matter behind. Examples of the bioabsorbable materials include polyglycolic acid, polylactic acid, polydioxane, polycaprolactone, glycolic acid/lactic acid polyester and the like.

A length of the suture body portion 10 (suture 100 for use in flexible endoscopic surgeries) can be changed as appropriate according to the size or the like of the opening portion, and is not particularly limited, but can be 10 cm or more, can be 13 cm or more, can be 15 cm or more, and can be 25 cm or less, for example.

A thickness of the suture body portion 10 can also be changed as appropriate according to a thickness or the like of the tissue in the vicinity of the opening portion, and is not particularly limited, but can be, for example, 0.15 mm or more, can be 0.20 mm or more, can be 0.30 mm or more, can be 0.35 mm or more, and can be 0.70 mm or less.

The thickness of the suture body portion 10 may be constant or may not be constant. When the thickness of the suture body portion 10 is not constant, the thickness of the suture body portion 10 can be made larger, for example, in a vicinity of the stop member 30 described later and in the center of the lengthwise direction of the suture body portion 10, than that of other portions of the suture body portion 10. By the configuration like this, loosening of the suture body portion 10 (suture 100 for use in flexible endoscopic surgeries) is more easily prevented.

As shown in Fig. 1, at the center in the lengthwise direction of the suture body portion 10, the one stop member 30 that prevents the suture body portion 10 from passing through is disposed. Note that the center in the lengthwise direction of the suture body portion 10 includes not only a position of 1/2 of the length of the suture body portion 10 but also a region located at 2/5 to 3/5 of the length of the suture body portion 10.

The stop member 30 functions as passing-through prevention of the suture body portion 10 (suture 100 for use in flexible endoscopic surgeries) on an outer wall side of tissue when both ends of the suture body portion 10 (suture 100 for use in flexible endoscopic surgeries) are respectively inserted from the outer wall side to the inner wall side of the tissue in the vicinity of the opening portion.

Conventionally, in the suture for use in endoscopic surgeries, a knot or the like has been made at one end portion, and used as passing-through prevention for the suture for use in the endoscopic surgeries. In the conventional suture for use in endoscopic surgeries, even when an end portion of the suture for use in endoscopic surgeries in which no passing-through prevention such as a knot is formed is inserted from a serous membrane or outer membrane side that is on the outer wall side of a stomach, intestine or the like to the inner wall side first, it is necessary to insert the end portion from the inner wall side of the stomach, intestine or the like to the serous membrane or outer membrane side that is on the outer wall side, next. Since other organs or the like exist on an outer side (direction on an opposite side to a direction normally visible by a lens of a flexible endoscope) of the serous membrane or outer membrane, it is necessary to pay the closest attention not to damage the other organs or the like when suturing using the conventional suture for use in endoscopic surgeries. However, in the suture 100 for use in flexible endoscopic surgeries according to the present embodiment, the one stop member 30 is disposed at the center in the lengthwise direction of the suture body portion 10, whereby each of both ends of the suture body portion 10 (suture 100 for use in flexible endoscopic surgeries) can be inserted from the serous membrane or outer membrane side that is on the outer wall side of the stomach, intestine or the like to the inner wall side of the stomach, intestine or the like in the vicinity of the opening portion, so that it is possible to more easily prevent the other organs or the like from being damaged. As a result, the suture 100 for use in flexible endoscopic surgeries according to the present embodiment can shorten a suturing time of the opening portion.

Further, according to the present invention, when each of both the ends of the suture body portion 10 (suture 100 for use in flexible endoscopic surgeries) is inserted, it is possible to confirm a distal end of each of both the ends of the suture body portion 10 (suture 100 for use in flexible endoscopic surgeries) with a video captured by the lens disposed in the distal end of the flexible endoscope, so that safety is excellent.

Although the stop member 30 is not particularly limited as long as the thickness of the stop member 30 is larger than the thickness of the suture body portion 10, and the stop member 30 functions as passing-through prevention of the suture body portion 10, the stop member 30 can have a size (thickness) larger than or equal to three times the thickness of the suture body portion 10, for example.

A shape of the stop member 30 is not particularly limited as long as the stop member 30 functions as the passing-through prevention of the suture body portion 10, and may also have a spherical shape, may have an elliptical shape, may have a semi-spherical shape, may have a semi-elliptical shape, may have a polygonal shape, may have a round column shape, may have a polygonal prism shape, may have a cylindrical shape, and may have a polygonal cylinder shape. Further, as described later, the stop member 30 may have a loop shape, may have a plate shape, may have a coil shape, may be a knot formed by tying the suture body portion 10, and may be a clip that can hold the suture body portion 10. Further, as described later, when the suture body portion 10 is composed of two suture pieces, a portion joining the two suture pieces may be the stop member 30.

Although the stop member 30 is not particularly limited, a maximum value of a width orthogonal to the lengthwise direction of the suture body portion 10 can be larger than a maximum value of a width parallel to the lengthwise direction of the suture body portion 10, for example. By the configuration like this, it is easy to enter between the serous membrane or the outer membrane and the serous membrane or the outer membrane of the tissue in the vicinity of the opening portion, and it is possible to hold the opening portion in a closed state and easily bring the tissue in the vicinity of the opening portion into a state of being raised to inside (inner wall side). In the stop member 30, a ratio of the maximum value of the width orthogonal to the lengthwise direction of the suture body portion 10, and the maximum value of the width parallel to the lengthwise direction of the suture body portion 10 (the maximum value of the width orthogonal to the lengthwise direction : the maximum value of the width parallel to the lengthwise direction) can be, for example, 1:1, can be 2:1, can be 3:1, can be 4:1, can be 5:1, can be 6:1, can be 7:1, can be 8:1, can be 9:1, can be 10:1, can be 12:1 can be 15:1, can be 20:1, can be 25:1, can be 30:1, can be 40:1, can be 50:1, can be 60:1, and can be 70:1. That is to say, in the stop member 30, the ratio of the maximum value of the width orthogonal to the lengthwise direction of the suture body portion 10, and the maximum value of the width parallel to the lengthwise direction of the suture body portion 10 can be, for example, 1:1 to 70:1, can be 2:1 to 60:1, and 3:1 to 50:1.

When the suture 100 for use in flexible endoscopic surgeries according to the present embodiment is used for full-thickness suture of a digestive tract, for example, the maximum value of the width orthogonal to the lengthwise direction of the suture body portion 10, of the stop member 30 is appropriately selected according to the thickness of the suture body portion 10, but can be, for example, 0.15 mm or more, can be 0.2 mm or more, can be 0.3 mm or more, can be 0.4 mm or more, can be 0.45 mm or more, can be 3 mm or less, can be 2.5 mm or less, can be 2 mm or less, can be 1.5 mm or less, and can be 1 mm or less.

Further, in the above-described case, the maximum value of the width parallel to the lengthwise direction of the suture body portion 10, of the stop member 30 is not particularly limited, but can be, for example, 0.15 mm or more, can be 0.2 mm or more, can be 0.3 mm or more, can be 0.4 mm or more, can be 0.45 mm or more, can be 10 mm or less, can be 9 mm or less, can be 8 mm or less, can be 7 mm or less, can be 6 mm or less, can be 5 mm or less, can be 4 mm or less, can be 3 mm or less, can be 2.5 mm or less, can be 2 mm or less, can be 1.5 mm or less, and can be 1 mm or less.

When the suture 100 for use in flexible endoscopic surgeries according to the present embodiment is used, for example, for full-thickness suture of a digestive tract, according to the above-described configuration, the stop member 30 easily enters between the serous membrane or outer membrane and the serous membrane or outer membrane of the tissue in the vicinity of the opening portion of the digestive tract, holds the opening portion in a closed state, and easily brings the tissue in the vicinity of the opening portion into a state raised to an inside (inner wall side), so that it is possible to insert the suture body portion 10 into a raised tissue portion, and it is possible to easily and completely close the opening portion of the digestive tract across all layers in a short period of time. Further, in the stop member 30 (plate-shape or the like described later, for example) that is formed so that the maximum value of the width orthogonal to the lengthwise direction of the suture body portion 10 is larger than the maximum value of the width parallel to the lengthwise direction of the suture body portion 10, the tissue in the vicinity of the opening portion is easily curved to the inside (inner wall side) (making it easier to be brought into a raised state) along the surface substantially orthogonal to the lengthwise direction of the suture body portion 10, of the stop member 30, so that the suturing time is shortened.

As shown in Fig. 1, the stop member 30 according to the present embodiment is configured to have a spherical shape. As a result of the stop member 30 having a spherical shape, as described later, when each of both the ends of the suture body portion 10 (suture for use in flexible endoscopic surgeries) is inserted from the outer wall side to the inner wall side of the tissue in the vicinity of the opening portion, and thereafter, tension is further applied from one side of the suture body portion 10 (suture 100 for use in flexible endoscopic surgeries), the stop member 30 easily enters between a serous membrane or an outer membrane and a serous membrane or an outer membrane of the tissue in the vicinity of the opening portion, holds the opening portion in a closed state, and easily brings the tissue in the vicinity of the opening portion into a state of being raised to inside (inner wall side), and the raised tissue portion can be made a target into which the suture body portion 10 is inserted, so that the time for suturing the opening portion can be shortened (see Fig. 6). Further, when a plurality of sutures 100 for use in flexible endoscopic surgeries are accommodated in an ordinary container, the plurality of sutures 100 for use in flexible endoscopic surgeries are hardly entangled with one another as a result that the stop member 30 has no corners and is hardly caught by the other sutures for use in flexible endoscopic surgeries.

As shown in Fig. 2, the suture body portion 10 includes a plurality of protrusion portions 50 that obliquely protrude toward an opposite side to the insertion direction of the suture body portion 10 (suture 100 for use in flexible endoscopic surgeries). As a result of including the plurality of protrusion portions 50 obliquely protruding toward the opposite side to the insertion direction of the suture body portion 10 (suture 100 for use in flexible endoscopic surgeries), it is possible to restrain the suture body portion 10 (suture 100 for use in flexible endoscopic surgeries) inserted into the tissue in the vicinity of the opening portion from moving or passing through in an anti-insertion direction on an opposite side to the insertion direction.

The number of the plurality of protrusion portions 50 is not particularly limited, and can be changed as appropriate, as long as they can prevent the suture body portion 10 (suture 100 for use in flexible endoscopic surgeries) inserted into the tissue in the vicinity of the opening portion from moving or passing through in the anti-insertion direction.

The plurality of protrusion portions 50 may be disposed at equal intervals or may be disposed at non-equal intervals. When the plurality of protrusion portions 50 are not disposed at equal intervals with respect to the lengthwise direction of the suture body portion 10, disposition intervals can be made narrower, for example, in the center in the lengthwise direction of the suture body portion 10, in the vicinity of the stop member 30, than in the other portions of the suture body portion 10. The configuration like this more easily prevents looseness of the suture body portion 10 (suture 100 for use in flexible endoscopic surgeries).

Inclination angles of the plurality of protrusion portions 50 are not particularly limited, and can also be changed as appropriate according to the thickness or the like of the tissue in the vicinity of the opening portion, as long as they can restrain the suture body portion 10 (suture 100 for use in flexible endoscopic surgeries) that is inserted into the tissue in the vicinity of the opening portion from moving or passing through in the anti-insertion direction, but can be 15° or less with respect to the suture body portion 10, for example.

The lengths of the plurality of protrusion portions 50 are not particularly limited, and can also be changed as appropriate according to the thickness or the like of the tissue in the vicinity of the opening portion, as long as they can restrain the suture body portion 10 (suture 100 for use in flexible endoscopic surgeries) inserted into the tissue in the vicinity of the opening portion from moving or passing through in the anti-insertion direction, but they can be, for example, more than or equal to 1/3 of the thickness of the suture body portion 10.

The thicknesses of the plurality of protrusion portions 50 are not particularly limited, and can also be changed as appropriate according to the thickness or the like of the tissue in the vicinity of the opening portion, as long as they can restrain the suture body portion 10 (suture 100 for use in flexible endoscopic surgeries) inserted into the tissue in the vicinity of the opening portion from moving or passing through in the anti-insertion direction.

The plurality of protrusion portions 50 include the plurality of first protrusion portions 51 that are disposed on one side, with the stop member 30 as the reference, in the lengthwise direction of the suture body portion 10, and obliquely protrude toward the other side and outside of the suture body portion 10, and the plurality of protrusion portions 52 that are disposed on the other side, with the stop member 30 as the reference, in the lengthwise direction of the suture body portion 10, and obliquely protrude toward the one side and outside of the suture body portion 10 (see Fig. 1 and Fig. 2).

As shown in the partial view (a) in Fig. 2, the plurality of first protrusion portions 51 that are disposed on the left side of the paper surface in Fig. 1, with the stop member 30 as the reference, in the lengthwise direction of the suture body portion 10 are configured to obliquely protrude in the anti-insertion direction (the right side of the paper surface in Fig. 1) and to outside (the upper side of the paper surfaces or the lower side of the paper surfaces in Fig. 1 and Fig. 2) of the suture body portion 10 (see Fig. 1). As shown in the partial view (b) in Fig. 2, the plurality of second protrusion portions 52 disposed on the right side of the paper surface in Fig. 1, with the stop member 30 as the reference, in the lengthwise direction of the suture body portion 10 are configured to obliquely protrude in the anti-insertion direction (the left side of the paper surface in Fig. 1) and to outside (the upper side of the paper surfaces or the lower side of the paper surfaces in Fig. 1 and Fig. 2) of the suture body portion 10 (see Fig. 1).

As a result of the suture 100 for use in flexible endoscopic surgeries including the first protrusion portions 51 and the second protrusion portions 52, it is possible to insert each of both the ends of the suture body portion 10 (suture 100 for use in flexible endoscopic surgeries) smoothly from the outer wall side to the inner wall side of the tissue in the vicinity of the opening portion, and prevent the suture body portion 10 (suture 100 for use in flexible endoscopic surgeries) from moving or passing through in the anti-insertion direction.

In more detail, it is possible to insert one end of the suture body portion 10 (suture 100 for use in flexible endoscopic surgeries) smoothly from the outer wall side to the inner wall side of the tissue in the vicinity of a spot where an opening diameter of the opening portion is maximum (see Fig. 3), and it is possible to insert the other end of the suture body portion 10 (suture 100 for use in flexible endoscopic surgeries) smoothy from the outer wall side to the inner wall side of the tissue in the vicinity of the opening portion located on an opposite side to the spot where the one end of the suture body portion 10 (suture 100 for use in flexible endoscopic surgeries) is inserted (see Fig 4) in a spot where an opening diameter of the opening portion is maximum. Then, it is possible to prevent the inserted suture body portion 10 (suture 100 for use in flexible endoscopic surgeries) from moving or passing through in the anti-insertion direction. Accordingly, as described above, it is possible to more easily prevent other external organs or the like from being damaged. As a result, the suture 100 for use in flexible endoscopic surgeries according to the present embodiment can shorten the time for suturing the opening portion. Further, since it is possible to confirm the distal ends of the one end and the other end of the suture body portion 10 (suture 100 for use in flexible endoscopic surgeries) with a video taken by the lens disposed in the distal end of the flexible endoscope, excellent safety is exhibited.

The configuration example of the suture 100 for use in flexible endoscopic surgeries according to the first embodiment is described above. Next, a method of using the suture 100 for use in flexible endoscopic surgeries according to the first embodiment will be described by using Fig. 3 to Fig. 8. Here, Fig. 3 is a view explaining a state in which the one end of the suture for use in flexible endoscopic surgeries according to the first embodiment is inserted from the outer wall side to the inner wall side of the tissue in the vicinity of the opening portion. Fig. 4 is a view explaining a state in which after the one end of the suture for use in flexible endoscopic surgeries according to the first embodiment is inserted from the outer wall side to the inner wall side of the tissue in the vicinity of the opening portion, the other end of the suture for use in flexible endoscopic surgeries is inserted into the vicinity of the tissue located on an opposite side to the tissue of the opening portion where the one end of the suture for use in flexible endoscopic surgeries is inserted from the outer wall side to the inner wall side. Fig. 5 is a view for explaining a state after both the ends of the suture for use in flexible endoscopic surgeries according to the first embodiment are inserted from the outer wall side to the inner wall side of the tissue in the vicinity of the opening portion. Fig. 6 is a sectional view for explaining a state in which after both the ends of the suture for use in flexible endoscopic surgeries according to the first embodiment are inserted from the outer wall side to the inner wall side of the tissue in the vicinity of the opening portion, tension is further applied from one side of the suture for use in flexible endoscopic surgeries. Fig. 7 is a sectional view for explaining a state in which one end of the suture for use in flexible endoscopic surgeries is inserted into the tissue that is raised by inserting both the ends of the suture for use in flexible endoscopic surgeries according to the first embodiment from the outer wall side to the inner wall side of the tissue in the vicinity of the opening portion and applying tension from one side. Fig. 8 is a view for explaining a state in which suturing of the opening portion is completed by the suture for use in flexible endoscopic surgeries according to the first embodiment. Note that in Fig. 3 to Fig. 8, the sizes of the plurality of protrusion portions 50 (51, 52) are increased for convenience of explanation.

In order to suture an opening portion OP by the suture 100 for use in flexible endoscopic surgeries according to the first embodiment, it is possible to use a gripper T that grips the suture 100 for use in flexible endoscopic surgeries as shown in Fig. 3. The gripper T is not particularly limited as long as it can be put in and taken out from a forceps hole disposed in the distal end of the flexible endoscope, and can grip the suture 100 for use in flexible endoscopic surgeries (or a needle included by a suture unit for use in flexible endoscopic surgeries described later).

In the suture 100 for use in flexible endoscopic surgeries, one end of the suture body portion 10 (suture 100 for use in flexible endoscopic surgeries) can be inserted into, for example, the tissue in the vicinity of the spot where the opening diameter of the opening portion OP is maximum from the outer wall side of a stomach, intestine or the like to the inner wall side, while gripping the suture body portion 10 (suture 100 for use in flexible endoscopic surgeries) on one side with the stop member 30 as the reference by the gripper T (see Fig. 3). At this time, as shown in Fig. 3, the plurality of protrusion portions 50 (51) are configured to be disposed at the suture body portion 10 (suture 100 for use in flexible endoscopic surgeries) on one side with the stop member 30 as the reference, and therefore it is possible to restrain the suture body portion 10 (suture 100 for use in flexible endoscopic surgeries) from moving or passing through in the anti-insertion direction. Consequently, according to the present invention, it is possible to smoothly advance an insertion operation of the suture body portion 10 (suture 100 for use in flexible endoscopic surgeries) into the tissue in the vicinity of the opening portion OP, and the suturing time is shortened. Note that insertion of the suture body portion 10 (suture 100 for use in flexible endoscopic surgeries) is started from the tissue in the vicinity of the opening portion OP on the left side of the paper surface in Fig. 3, but insertion of the suture body portion 10 (suture 100 for use in flexible endoscopic surgeries) may be started from the tissue in the vicinity of the opening portion OP on the right side of the paper surface in Fig. 3, and an order thereof is not particularly limited.

Next, as shown in Fig. 4, in the suture 100 for use in flexible endoscopic surgeries, the other end of the suture body portion 10 (suture 100 for use in flexible endoscopic surgeries) can be inserted into the tissue located on the opposite side to the tissue of the opening portion OP where the one end of the suture body portion 10 (suture 100 for use in flexible endoscopic surgeries) is inserted, from the outer wall side of a stomach, an intestine or the like to the inner wall side, while gripping the suture body portion 10 (suture 100 for use in flexible endoscopic surgeries) on the other side with the stop member 30 as the reference with the gripper T. Since the plurality of protrusion portions 50 (52) are configured to be disposed in the suture body portion 10 (suture 100 for use in flexible endoscopic surgeries) on the other side with the stop member 30 as the reference, it is possible to restrain the suture body portion 10 (suture 100 for use in flexible endoscopic surgeries) from moving or passing through in the anti-insertion direction when the suture body portion 10 (suture 100 for use in flexible endoscopic surgeries) is inserted into the tissue in the vicinity of the opening portion OP. Consequently, according to the present invention, it is possible to smoothly advance the insertion operation of the suture body portion 10 (suture 100 for use in flexible endoscopic surgeries) into the tissue in the vicinity of the opening portion OP, and suturing time is shortened.

The suture 100 for use in flexible endoscopic surgeries according to the present embodiment can hold the opening portion OP in a closed state as shown in Fig. 5, because when both the ends of the suture body portion 10 (suture 100 for use in flexible endoscopic surgeries) are inserted from the outer wall side to the inner wall side of a stomach, an intestine or the like, and tension is applied to the inserted suture body portion 10 (suture 100 for use in flexible endoscopic surgeries), the stop member 30 and the plurality of protrusion portions 50 (51, 52) cooperate to restrain movement in the anti-insertion direction of the inserted suture body portion 10 (suture 100 for use in flexible endoscopic surgeries), and passing-through of the inserted suture body portion 10 (suture 100 for use in flexible endoscopic surgeries).

When tension is further applied by pulling the suture body portion 10 (suture 100 for use in flexible endoscopic surgeries) from one side from the state shown in Fig. 5, the stop member 30 cooperates with the plurality of protrusion portions 50 (51, 52) to hold the opening portion OP in the closed state on the outer wall side, and can bring the tissue in the vicinity of the opening portion OP in the state of being raised to inside (inner wall side, that is, a direction normally visible by the lens of the flexible endoscope) as shown in Fig. 6.

When both the ends of the suture body portion 10 (suture 100 for use in flexible endoscopic surgeries) are inserted into the tissue in the vicinity of the opening portion OP, and the tissue in the vicinity of the opening portion OP is in the raised state, each of both the ends of the suture body portion 10 (suture 100 for use in flexible endoscopic surgeries) can be thereafter inserted into the raised tissue portion as shown in Fig. 7. In more detail, when the suture body portion 10 (suture 100 for use in flexible endoscopic surgeries) is inserted into the portion where the tissue in the vicinity of the opening portion OP is raised, there is a low possibility of damaging other organs or the like, and therefore, the suture body portion 10 (suture 100 for use in flexible endoscopic surgeries) can be inserted at one time (or at once) from the inner wall side of a stomach, an intestine or the like through the outer wall to the inner wall side on the opposite side. Therefore, the suture body portion 10 (suture 100 for use in flexible endoscopic surgeries) can be easily inserted into all layers composing the stomach, intestine or the like.

Inserting the suture body portion 10 (suture 100 for use in flexible endoscopic surgeries) to the tissue in the vicinity of the opening portion OP that is raised is easier than inserting the suture body portion 10 (suture 100 for use in flexible endoscopic surgeries) to the tissue in the vicinity of the opening portion OP that is only closed, because there is less movement of the gripper T and the suture 100 for use in flexible endoscopic surgeries gripped by the gripper T, continuous insertion is possible, and the suture 100 can be operated within the range displayed in the image captured by the lens installed in the distal end of the flexible endoscope. Consequently, the suture 100 for use in flexible endoscopic surgeries according to the present embodiment can shorten the time for suturing the opening portion OP.

When each of both the ends of the suture body portion 10 (suture 100 for use in flexible endoscopic surgeries) is inserted several times into the tissue in the vicinity of the opening portion OP that is raised, suturing of the opening portion OP is completed as shown in Fig. 8. In this way, according to the suture 100 for use in flexible endoscopic surgeries according to the present embodiment, it is possible to close the opening portion OP easily and completely.

In the suture 100 for use in flexible endoscopic surgeries according to the present embodiment, one end of the suture body portion 10 is inserted from the outer wall side to the inner wall side of the tissue in the vicinity of the opening portion OP of human or animal tissue, for example, in the vicinity of the opening portion OP where the opening diameter of the opening portion OP is maximum, the other end of the suture body portion 10 is inserted from the outer wall side to the inner wall side of the tissue located on the opposite side to the tissue of the opening portion OP where the one end of the suture body portion 10 is inserted, and thereafter tension is applied, whereby the stop member 30 cooperates with the plurality of protrusion portions 50 (51, 52) to hold the opening portion in the closed state on the outer wall side, and brings the tissue in the vicinity of the opening portion OP into the state of being raised to inside (inner wall side, that is, the direction normally visible by the lens of the flexible endoscope), and each of both the ends of the suture body portion 10 can be continuously inserted into the raised portion (full-thickness continuous suture).
Accordingly, the suture 100 for use in flexible endoscopic surgeries according to the present embodiment is favorably used in the suturing method of flexible endoscopic surgeries. The suturing method of the flexible endoscopic surgery can more easily prevent other external organs or the like from being damaged, and as a result, can shorten the time for suturing the opening portion. Further, since distal ends of one end and the other end of the suture body portion 10 (suture 100 for use in flexible endoscopic surgeries) can be confirmed by the video taken with the lens disposed in the distal end of the flexible endoscope, excellent safety is exhibited. That is to say, according to the present invention, the suturing method in flexible endoscopic surgeries using the suture for use in flexible endoscopic surgeries or a suture unit for use in flexible endoscopic surgeries described later is also provided.

The suture 100 for use in flexible endoscopic surgeries according to the present embodiment is described above. Next, a suture 120 for use in flexible endoscopic surgeries according to a second example will be described by using Fig. 9 to Fig. 11. Here, Fig. 9 is a view for explaining a configuration example of a suture for use in flexible endoscopic surgeries according to the second embodiment. Fig. 10 is a view showing an example of a method for manufacturing the suture for use in flexible endoscopic surgeries according to the second embodiment, a partial view (a) in Fig. 10 is a view illustrating configurations of a first suture piece and a second suture piece, and a partial view (b) in Fig. 10 is a view illustrating a state in which the first suture piece and the second suture piece are joined to each other. Fig. 11 is a view showing an example of another method for manufacturing the suture for use in flexible endoscopic surgeries according to the second embodiment, a partial view (a) in Fig. 11 is a view illustrating a state in which the second suture piece is inserted into a large diameter portion of the first suture piece, and the first suture is inserted into a large diameter portion of the second suture piece, and a partial view (b) in Fig 11 is a view illustrating a state in which the large diameter portion of the first suture piece and the large diameter portion of the second suture piece are joined to each other. Note that the same components as or similar components to those in the aforementioned embodiment are assigned with the same reference signs and explanation thereof may be omitted.

### [Suture 120 for Use in Flexible Endoscopic Surgeries According to Second Embodiment]

As shown in Fig. 9, a suture 120 for use in flexible endoscopic surgeries according to a second embodiment can be configured such that a suture body portion 10 includes a first suture piece 11 having a plurality of protrusion portions 50, and a second suture piece 13 having a plurality of protrusion portions 50, and a stop member 30 is disposed in a joining portion of the first suture piece 11 and the second suture piece 13.

The suture body portion 10 according to the present embodiment can include the first suture piece 11 and the second suture piece 13. A joining method of the first suture piece 11 and the second suture piece 13 is not particularly limited.

As shown in the partial view (a) in Fig. 10, the first suture piece 11 according to the present embodiment includes a first suture piece body portion 12, the plurality of protrusion portions 50 (51), and a large diameter portion 21 having a larger thickness or size than the first suture piece body portion 12 at one end thereof.

The large diameter portion 21 of the first suture piece 11 has a loop shape, but a shape thereof is not particularly limited, and may have a shape illustrated as a shape of the stop member 30 described above. Further, the thickness or the size of the large diameter portion 21 of the first suture piece 11 is not particularly limited as long as it is larger than the first suture piece body portion 12, and becomes a passing-through prevention of the suture body portion 10 (suture 120 for use in flexible endoscopic surgeries) when it is joined to the second suture piece 13 to be a joining portion 31.

As shown in the partial view (a) in Fig 10, the second suture piece 13 according to the present embodiment includes a second suture piece body portion 14, the plurality of protrusion portions 50 (52), and a large diameter portion 21 having a larger thickness or size than the second suture piece body portion 14 at one end thereof. A shape, the thickness, size, and the like of the large diameter portion 21 of the second suture piece 13 are not particularly limited as long as it becomes passing-through prevention of the suture body portion 10 (suture 120 for use in flexible endoscopic surgeries) when it is joined to the first suture piece 11 to be the joining portion 31 similarly to the large diameter portion 21 of the first suture piece 11.

As shown in Fig. 10, in the present embodiment, the large diameter portion 21 of the first suture piece 11, and the large diameter portion 21 of the second suture piece 13 are joined to each other, and thereby the large diameter portions 21 and 21 are used as the joining portion 31, and as the stop member 30. A joining method of the first suture piece 11 and the second suture piece 13 are not particularly limited, and they may be joined by using an adhesive or the like.

Further, since the large diameter portion 21 according to the present embodiment has a loop shape, the second suture piece 13 is inserted into the loop of the large diameter portion 21 of the first suture piece 11, and the first suture piece 11 is inserted into the loop of the large diameter portion 21 of the second suture piece 13 as shown in the partial view (a) in Fig. 11, whereby the first suture piece 11 and the second suture piece 13 are joined to each other, and the stop member 30 may be formed as shown in the partial view (b) in Fig. 11.

The stop member 30 according to the present embodiment is configured to have the loop shape, but the shape thereof is not particularly limited as described above.

In this way, the suture 120 for use in flexible endoscopic surgeries according to the present embodiment is formed by combining the two suture pieces 11 and 13. When the two suture pieces 11 and 13 are combined, the plurality of protrusion portions 50 are disposed to obliquely protrude toward an opposite side to the insertion direction, as shown in Fig. 9 to Fig. 11.

The sutures 100 and 120 for use in flexible endoscopic surgeries according to the aforementioned embodiments can be a suture unit for use in flexible endoscopic surgeries including needles at both ends of the suture body portion 10. In other words, the suture unit for use in flexible endoscopic surgeries uses the sutures 100 and 120 for use in flexible endoscopic surgeries including needles at both ends of the suture body portion 10.

As the needles disposed at both the ends of the suture body portion 10, needles used in flexible endoscopic surgeries can be used, and can have a curved shape, for example. When the needles have a curved shape, a diameter of the needle can be changed as appropriate according to the thickness of the tissue in the vicinity of the opening portion, for example, and can be larger than or equal to twice the thickness of the tissue in the vicinity of the opening portion.

Although the preferred embodiments of the present invention are described above, the technical scope of the present invention is not limited to the range described in the above-described embodiments. Various changes or alterations can be added to the above-described embodiments.

For example, in the aforementioned embodiments, the stop member 30 is configured to have a spherical shape or the loop shape, but may be a plate-shape. The plate-shaped stop member 30 includes a through-hole where the suture body portion 10 is passed in a center of a plate-shaped member, for example, and can be manufactured by passing the suture body portion 10 in the through-hole, or integrally molding the suture body portion 10 and the stop member 30.

As shown in a partial view (a) in Fig. 12, for example, the stop member 30 can have a plate shape having a wall surface formed in a direction substantially orthogonal to the lengthwise direction of the suture body portion 10, and an end surface formed in a direction substantially parallel to the lengthwise direction of the suture body portion 10, and formed so that a maximum value of a width orthogonal to the lengthwise direction of the suture body portion 10 becomes larger than a maximum value of a width parallel to the lengthwise direction of the suture body portion 10. By the configuration like this, as shown in the partial view (b) in Fig. 12, the plate-shaped stop member 30 easily enters between a serous membrane or an outer membrane and a serous membrane or an outer membrane of the opening portion, and easily functions to prevent the suture body portion 10 from passing through in cooperation with the plurality of protrusion portions 50 (51, 52). Further, since the tissue in the vicinity of the opening portion is easily curved to inside (inner wall side) along the wall surface (easily brought into a raised state), the suturing time is shortened.

As shown in the partial view (a) in Fig. 13, the stop member 30 can be formed into a coil shape. As shown in the partial view (a) in Fig. 13, the stop member 30 having a coil shape can be formed by, for example, winding the suture body portion 10 a plurality of times in a portion at the center in the lengthwise direction of the suture body portion 10, and joining the portion of the coil shape (stop member 30) by an adhesive or the like, for example. Further, the stop member 30 can also be manufactured by, for example, passing the suture body portion 10 into a member having a coil shape and made of a bioabsorbable material, and joining it by an adhesive or the like. Further, for example, around the suture body portion 10, another suture body portion 10 may be wound a plurality of times to form a coil shape, and in that case, the other suture body portion 10 that is wound the plurality of times can be joined by an adhesive or the like, for example.

Furthermore, for example, the stop member 30 may be a knot formed by tying the suture body portion 10.

Furthermore, as shown in the partial view (b) in Fig. 13, for example, the stop member 30 may be a clip that can hold the suture body portion 10. As shown in the partial view (b) in Fig. 13, the stop member 30 may be formed by holding the one suture body portion 10 with the clip, or as shown in the partial view (c) in Fig. 13, the stop member 30 may be formed by holding the two suture pieces 11 and 13 at the same time. Note that the clip that holds the suture body portion 10 (suture pieces 11 and 13) can be made of a bioabsorbable material.

Further, in the aforementioned embodiment, the large diameter portion 21 of the first suture piece 11 and the large diameter portion 21 of the second suture piece 13 are each configured to have a single loop shape, but, for example, as shown in Fig. 14, the large diameter portion 21 may be configured by winding an end portion that is not a side inserted into human or animal tissue in a loop shape (coil shape) in each of the first suture piece 11 (first suture piece body portion 12) and the second suture piece 13 (second suture piece body portion 14). In this case, the end portions of the first suture piece 11 (first suture piece body portion 12) and the second suture piece 13 (second suture piece body portion 14) can be configured to be disposed on the loop shape (coil shape) portions. According to the configuration like this, the protrusion portions 50 (51, 52) can be formed to the vicinity of the large diameter portion 21, as compared with the case where the end portions of the first suture piece 11 (first suture piece body portion 12) and the second suture piece 13 (second suture piece body portion 14) are disposed at the first suture piece 11 (first suture piece body portion 12) and the second suture piece 13 (second suture piece body portion 14). In the first suture piece 11 and the second suture piece 13 as above, the stop member 30 may be formed by using the large diameter portions 21 and 21 as the joining portion, and joining the large diameter portion 21 of the first suture piece 11 and the large diameter portion 21 of the second suture piece by an adhesive or the like, for example (see Fig. 10) as in the aforementioned embodiment. Further, by inserting the second suture piece 13 into the loop of the large diameter portion 21 of the first suture piece 11, and inserting the first suture piece 11 into the loop of the large diameter portion 21 of the second suture piece 13, the first suture piece 11 and the second suture piece 13 may be joined, and the large diameter portions 21 and 21 may be used as the joining portion to form the stop member 30 (see Fig. 11).

Further, the first suture piece body portion 12 and the second suture piece body portion 14 may be each composed of a single thread similarly to the suture body portion 10 or may be multiple overlapping threads such as double threads.

### REFERENCE SIGNS LIST

100, 120: suture for use in flexible endoscopic surgeries
10: suture body portion, 11: first suture piece, 12: first suture piece body portion, 13: second suture piece, 14: second suture piece body portion
21: large diameter portion
30: stop member, 31: joining portion
50 (51, 52): protrusion portion, 51: first protrusion portion, 52: second protrusion portion OP: opening portion

## Claims

1. A suture for use in flexible endoscopic surgeries for suturing an opening portion of human or animal tissue, comprising:
a suture body portion;
one stop member that is disposed at a center in a lengthwise direction of the suture body portion and prevents the suture body portion from passing through; and
a plurality of protrusion portions that obliquely protrude toward an opposite side to an insertion direction of the suture body portion,
wherein the plurality of protrusion portions comprise
a plurality of first protrusion portions that are disposed on one side, with the stop member as a reference, in the lengthwise direction of the suture body portion, and protrude obliquely toward another side and outside of the suture body portion, and
a plurality of second protrusion portions that are disposed on the other side, with the stop member as the reference, in the lengthwise direction of the suture body portion, and protrude obliquely toward the one side and outside of the suture body portion.

2. The suture for use in flexible endoscopic surgeries according to claim 1, wherein both ends of the suture body portion are inserted from an outer wall side to an inner wall side of tissue in a vicinity of the opening portion.

3. The suture for use in flexible endoscopic surgeries according to claim 1, wherein one end of the suture body portion is inserted from an outer wall side to an inner wall side of tissue in a vicinity of the opening portion where an opening diameter of the opening portion is maximum, and another end of the suture body portion is inserted from an outer wall side to an inner wall side of tissue located on an opposite side to the tissue of the opening portion where the one end of the suture body portion is inserted.

4. The suture for use in flexible endoscopic surgeries according to claim 1, wherein the stop member holds the opening portion in a closed state on an outer wall side in cooperation with the plurality of protrusion portions.

5. The suture for use in flexible endoscopic surgeries according to claim 1, wherein
the suture body portion comprises a first suture piece having the plurality of protrusion portions, and a second suture piece having the plurality of protrusion portions, and
the stop member is disposed at a joining portion of the first suture piece and the second suture piece.

6. The suture for use in flexible endoscopic surgeries according to claim 1, wherein the stop member has a spherical shape.

7. The suture for use in flexible endoscopic surgeries according to claim 1, wherein the stop member is plate-shaped.

8. The suture for use in flexible endoscopic surgeries according to claim 1, wherein the stop member has a coil shape.

9. The suture for use in flexible endoscopic surgeries according to claim 1, wherein the stop member is a knot formed by tying the suture body portion.

10. The suture for use in flexible endoscopic surgeries according to claim 1, wherein the stop member is a clip that can hold the suture body portion.

11. The suture for use in flexible endoscopic surgeries according to claim 1, comprising a bioabsorbable material.

12. A suture unit for use in flexible endoscopic surgeries using the suture for use in flexible endoscopic surgeries according to any one of claims 1 to 11, comprising needles at both ends of the suture body portion.
